Europäisches Patentamt

European Patent Office

Office européen des brevets

(11) Publication number: **0 083 371**
**B1**

(12) **EUROPEAN PATENT SPECIFICATION**

(45) Date of publication of patent specification: **22.10.86**

(51) Int. Cl.⁴: **A 61 L 15/03**

(21) Application number: **82902506.3**

(22) Date of filing: **08.07.82**

(86) International application number:
**PCT/US82/00928**

(87) International publication number:
**WO 83/00093 20.01.83 Gazette 83/02**

(54) **TRINITROGLYCEROL SUSTAINED RELEASE VEHICLES AND PREPARATION THEREFROM.**

(30) Priority: **08.07.81 US 281389**

(43) Date of publication of application:
**13.07.83 Bulletin 83/28**

(45) Publication of the grant of the patent:
**22.10.86 Bulletin 86/43**

(84) Designated Contracting States:
**AT BE CH DE FR GB LI LU NL SE**

(56) References cited:
**EP-A-0 013 606**
**DE-B-1 209 034**
**FR-A-1 409 082**
**GB-A-1 090 184**
**GB-A-1 127 881**
**GB-A-2 021 610**
**US-A-2 155 658**
**US-A-2 693 438**
**US-A-3 287 222**
**US-A-3 429 308**
**US-A-3 742 951**
**US-A-3 789 119**
**US-A-3 972 995**

(73) Proprietor: **Key Pharmaceuticals, Inc.**
**4400 Biscayne Boulevard**
**Miami, Florida 33137 (US)**

(72) Inventor: **KEITH, Alec Dell**
**18425 N.W. 2nd Avenue**
**Miami, FL 33169 (US)**
Inventor: **SNIPES, Wallace**
**P.O. Box 298**
**Pine Grove Mills, PA 16801 (US)**

(74) Representative: **Werner, Hans-Karsten, Dr. et al**
**Deichmannhaus am Hauptbahnhof**
**D-5000 Köln 1 (DE)**

(56) References cited:
**US-A-4 059 686**
**US-A-4 091 091**
**US-A-4 210 633**
**US-A-4 226 848**
**US-A-4 291 015**
**US-A-4 292 299**
**US-A-4 336 243**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

(56) References cited:
No relevant documents have been disclosed.
Chemical Abstracts, Vol. 64, Issued 1966,
(Columbus, Ohio, USA), see Abstract 11022B of
Ger. 1, 209, 034, Jan. 13, 1966, DYNAMIT-
NOBEL A/6, LINGENS et al.

Chemical Abstracts, Vol. 65, Issued 1966,
(Columbus, Ohio, USA), See Abstract 65: 568B
of Fr. 1,409,082, Aug. 20, 1965, DYNAMIT-
NOBEL A/6

Chemical Abstracts, Vol. 58, Issued 1963,
(Columbus, Ohio, USA), See Abstract 58:
2317h- KOGYO KAYAKU KYOKAISHI 22: 254-8
(1961) "GELATINIZATION PROPERTIES OF
NITRO CELLULOSE AND NITROGLYCOLGELS"
AMERICAN PHARMACY VOL. N522, No. 2,
Published Feb. 1982, Pages 29-35, DASTA et al
"PROBING THE NITROGLYCERIN THERAPIES,
TOPICAL NITROGLYCERIN"

Chemical Abstracts, Vol. 94, Issued 1981,
(Columbus, Ohio, USA), See page 311, column
2, Abstract 94: 20412e, KEITH et al (KEY
PHARMACEUTICALS, INC.), EUR. PAT. APPL.
13606, 23 July 1980, "POLYMERIC DIFFUSION
MATRIX AND DRUG DELIVERY DEVICE
COMPRISING SAID MATRIX"

Chemical Abstracts, Vol. 83, Issued 1975,
(Columbus, Ohio, USA), See page 338, Abstract
83: 152389,of Fr. Addn. 2245161 18 April 1975,
ORYSMONDE, "EXCIPIENT FOR POMADES,
PASTES, AND CREAMS"

Chemical Abstracts, Vol. 79, Issued 1973,
(Columbus, Ohio, USA), See page 215, Abstract
79: 45845b of USSR 219116, VISHNEVSKII et al,
"PROTECTIVE AND MEDICATED FILM FOR THE
INITIAL TREATMENT OF SCALDS"

## Description

The invention provides an improved transdermal diffusion matrix for the sustained release of trinitroglycerol. The anti-anginal activity of trinitroglycerol has been well known for many years, and a sustained release of trinitroglycerol through the skin for systemic activity has been known for several decades, as evidenced by Davis et al, Am. J. Med. Sci., 259—263 (Sept. 1955). Nitrol (Kremers-Urban) and Nitro-Bid (Marion) have been on the market for some time. Gross et al, *Archiv für Toxikologie*, vol. 18, 194—199, 331—334 (1960), has noted the sustained release of trinitroglycerol in studies which confirm that trinitroglycerol is delivered through the skin at a steady rate. The rate determining step for trinitroglycerol systems is controlled by the skin itself. Around 1970, various sustained release forms were proposed as exemplified by Zaffaroni, U.S. Patent No. 3,942,751, although none of the embodiments disclosed were put into use. Recently, Nitro-Dur (Key Pharmaceuticals, Inc., Miami, Florida; Keith et al, U.S. Patent 4,291,015) has received favorable commercial introduction, and two other dissimilar products for the same use have also been proposed for commercial introduction (Searle; Ciba).

The invention provides a substantially disaccharide-free polymeric diffusion matrix for the transdermal systemic delivery of trinitroglycerol through the skin of a patient, said polymeric diffusion matrix containing sufficient trinitroglycerol to be released over a prolonged period of time and which comprises a first lower molecular weight water soluble polymer with hydration sites, a second higher molecular weight water soluble polymer with hydration sites and glycerol. Accordingly the present application is directed to a trinitroglycerol containing polymeric diffusion matrix for the transdermal systemic delivery of trinitroglycerol through the skin of a patient, said polymeric diffusion matrix containing sufficient trinitroglycerol to be released over a prolonged period of time, characterized in that it is substantially disaccharide-free and comprises 10 to 40 percent by weight of a first lower molecular weight, partial hydrolyzed polyvinylalcohol component with a molecular weight of from 5.000 to 40.000 and with a degree of hydrolysis of 75 to 92 percent and 2 to 15 percent by weight of a second higher molecular weight, essentially fully hydrolyzed polyvinylalcohol component with a molecular weight of from 90.000 to 150.000 and with a degree of hydrolysis of at least 95 percent and 2 to 25 percent by weight of glycerol.

The lower molecular weight polyvinylalcohol component as used herein is always a partially hydrolyzed form with a degree of hydrolysis of 75 to 92%, preferably between 86 and 90%. As used in all examples herein, the degree of hydrolysis is 88%. The higher molecular weight polyvinylalcohol component generally has at least 95% hydrolysis and preferably at least about 98% hydrolysis. When used in the examples, the degree of hydrolysis is about 98—99%.

In a further embodiment, the substantially disaccharide-free matrix includes polyvinylpyrrolidone in an amount up to about 2% by weight having a molecular weight of from 20,000 to 60,000.

The matrix may also include an alkanolamide present in an amount up to 5% by weight and preferably from 0.1 to 3% by weight.

The invention provides alternative forms for the sustained release of trinitroglycerol which do not require the presence of lactose. Up to the present time, lactose has generally been included in trinitroglycerol preparations because lactose triturate is the form in which trinitroglycerol has generally been available to the pharmaceutical industry. (It should be recognized that trinitroglycerol in concentrated form is highly explosive, which is the reason for a general requirement heretofore of the lactose triturate form).

Nitro-Dur is sustained release preparation that has been favorably received in commercial use. The polymeric diffusion matrix in accordance with the invention is superior to Nitro-Dur in terms of being less "wet" which, quite apart from the subjective aspects for a wearer contributes to minimizing the debonding of the adhesive, enabling a prolonged wearing by a user. The improved matrix promotes the intimate adherence throughout this prolonged period of the polymeric diffusion matrix to the skin, intimate diffusional contact being desired for the sustained release of the drug to the patient.

According to this invention, a disaccharide-free polymeric diffusion matrix is provided. The polymeric diffusion matrix is suitable for systemic trinitroglycerol delivery through the skin of a patient. In a preferred embodiment, the trinitroglycerol is added as a trinitroglycerol/solvent solution. The solvents used include polyethylene glycol, dipropylene glycol, diacetin and acetin. The amount of solvent ranges from about 5% to about 20%.

The matrix comprises from 10 to 40% lower molecular weight water soluble polymer, for example polyvinylalcohol, and preferably from 25 to 30% by weight; from 2 to 15% higher molecular weight water soluble polymer, and preferably about 3%; and from 2 to 25% glycerol, preferably from 10 to 15% by weight. The molecular weight of the lower molecular weight polyvinylalcohol is from 5,000 to 40,000, preferably from 20,000 to 23,000. The higher molecular weight water soluble polymer component is from 90,000 to 150,000, and preferably about 115,000.

In a further embodiment, the polymeric diffusion matrix includes polyvinylpyrrolidone in an amount up to about 2% by weight having a molecular weight from 20,000 to 60,000.

The matrix of the invention also includes an alkanolamide in an amount up to about 5%, preferably 0.1 to 3% by weight. These surface active agents are $C_8$—$C_{18}$ alkanolamides, for

example, lauric myristic, lauric, myristic, isostearic, capric, linoleic and combinations thereof. The alkanolamide can be, for example, Monamid 716 (Mona), Monamid 716P (Mona), Monateric LMAB (Mona), or Monaquat PTL (Mona). The above agents exemplify the properties desired in the surface active agents.

A lactose-free triturate, which is suitable as a means for providing trinitroglycerol when it is not desired to have lactose in the preparation may be used. It is to be understood that mixtures of the novel triturate without lactose may, of course, be used in combination with lactose triturate when desired for a particular use, the exemplifications of the invention which follow showing only the lactose-free formulations.

Trinitroglycerol in a stable form is provided by binding the trinitroglycerol to a polymeric substance which preferably is a lower molecular weight water soluble polymer, polyvinylpyrrolidone or a mixture of these polymers. With a lower molecular weight polyvinylalcohol of from 2,000 to 15,000 molecular weight, and still more preferably of about 10,000 molecular weight, a solvent solution of the trinitroglycerol may be poured into the polyvinylalcohol to achieve the triturate without heating. Typically, the trinitroglycerol is leached from lactose triturate with a solvent and the solvent containing the trinitroglycerol is poured into the polyvinylalcohol, the solvent subsequently evaporated to yield the desired trinitroglycerol bound to the polyvinylalcohol. A similar preparation of trinitroglycerol may be made with other polymers such as polyvinylpyrrolidone having a molecular weight of from 20,000 to 90,000. The amount of trinitroglycerol is not critical, provided that phase separation is avoided (which phase separation would lead to the risk of a dangerous explosion). Up to 20% trinitroglycerol has been experimentally introduced into a polyvinylalcohol preparation, although more generally a 10% trinitroglycerol preparation has been produced.

It should also be noted that it is expected that, when commercial demand for the polymeric triturate is sufficient, the preparation of the polymeric triturates of the invention, while initially produced through the method described above from lactose triturate, will be replaced by procedures that will directly bind the trinitroglycerol to the polymeric substances without an intermediate lactose triturate step.

It is to be understood that other triturates may be formed. It is, of course, essential and very important from the standpoint of safety that sufficiently low concentrations of trinitroglycerol be present to be certain to avoid phase separation. It is to be understood that as a practical matter a maximum of about 20% (and usually only about 10%) trinitroglycerol is included in any of the triturates of the invention. Examples of triturates in the explosives field with higher percentages of nitroglycerin are found in Berthmann et al, British Patent No. 1,090,184, which discloses methods for manufacture of various triturates. It is to be understood that any pharmacologically acceptable triturate may be prepared, including triturates of the same polymers of Berthmann et al, of course with a much lower concentration of trinitroglycerol than the explosive levels of that patent. As such polymers may be mentioned polymethacrylic acid ester, polyvinyl acetate, ethyl acrylate-vinyl chloride copolymer (Astralon®) and polymethyl methacrylate.

It should be noted that a difference between the polymeric forms of trinitroglycerol of the invention and lactose triturate exists so that the two substances cannot be used completely interchangeably. The polymeric forms of the invention appear to provide products of superior mechanical properties as compared to products based upon lactose.

It should also be noted that in the embodiments which refer to a triturate or to a polymeric mixture of polymerically bound trinitroglycerol varying percentages of trinitroglycerol may be used. Unless otherwise specified, however, when no fixed percentage of trinitroglycerol has been mentioned, the amount is 10% of the particular polymer. As noted elsewhere, higher amounts have been prepared, including 20%, and are contemplated within the scope of this invention. (It again is to be cautioned that working with very high percentages of trinitroglycerol moves toward the danger of phase separation, which is to be avoided in any event due to the risk of explosion. Great caution is to be exercised by the reader when deviation is made from examples in a manner to increase percentages of trinitroglycerol that would bring about phase separation).

It is to be noted that a bandage backing is desirable for the polymeric diffusion matrix of the invention to provide diffusional contact with the skin. In a preferred embodiment, the polymeric diffusion matrix is affixed to a patient in the form of a bandage. This form provides direct adherence of the matrix to the skin of the patient. The barrier layer, a combination baseplate and facestock, comprises an inert backing material including metal foils and polyesters. The drug containing matrix, in its liquid state, is cast between the above described barrier layer and a coverstrip layer with a double faced adhesive. The barrier coverstrip includes inert materials such as metal foils and polyesters. A label may optionally be included for the rapid identification of drug and dose in emergency situations.

In a further embodiment the bandage includes a facestock layer with skin adhesive which comprises a foam, film-type, non-woven or vinyl tape with an acrylic, silicone, or rubber adhesive. Useful backings include, for example, Mylar® (polyethylene terephthalate, DuPont), polyethylene, and a variety of polyesters. Also include is a barrier baseplate of inert backing material such as metal foils or polyester films. It is to be noted that the bandage should contain at least one polyester or foil layer on both sides of the matrix to prevent evaporation. As previously noted, the liquid matrix is cast between barrier layers. A matrix anchor between the barrier baseplate and polymeric diffusion matrix comprises a synthetic or non-woven material layer, such as nylon or polyester film. Prior to administration the outermost layer, the barrier coverstrip, is pulled away and the matrix in bandage form is affixed to the patient.

As a further embodiment in the packaging of the present matrix, the backing layer may be a conventional backing layer such as is disclosed in Keith et al, U.S. Patent No. 4,291,015, particularly where the polymeric diffusion matrix is supported through an annular adhesive strip surrounding the polymeric diffusion matrix. The drug-containing diffusion matrix is placed in a cavity provided in an inert backing material. Useful backing materials include metal foils such as aluminum foil, polyolefins such as polyethylene and polypropylene, polyesters such as polyethylene terephthalate and polyamides such as nylon. The drug-containing diffusion matrix can be poured in its molten state into the cavity and permitted to cool. An adhesive layer is provided on the backing material surrounding the cavity. To prevent evaporative loss in the surface of the matrix, the adhesive layer and the matrix are sealed with a release layer. To use the device, the patient peels off the release layer and places the device in intimate contact with his skin. The exposed adhesive layer secures the device to the patient. A concentration gradient existing normal to the surface of the matrix and the patient's skin facilitates diffusion of the drug through the matrix into the patient's body. Thus, there is provided a device whereby a drug is delivered transdermally to a patient at a steady rate over a prolonged period of time. To apply the drug to the patient, the cover layer is peeled off. The exposed matrix is then taped onto a suitable portion of the patient's body.

The amounts of trinitroglycerol which are to be included in any of the dosage forms are similar to those which are used in the US-Patent No. 4,291,015. Generally, a worker skilled in the art will be aware of a desired delivery dosage and include an excess over that amount for any of the sustained release forms.

Disaccharide-free triturates that may be used to provide the trinitroglycerol for the polymeric diffusion matrix of the invention are described in the following examples:

Example I

A 10% trinitroglycerol triturate that is lactose-free is produced by leaching trinitroglycerol from lactose triturate with ether, and permitting the ether-bearing trinitroglycerol to be added into polyvinylalcohol (mw 10,000, 88% hydrolyzed). The ether mixture is first filtered to remove lactose before being added to the polyvinylalcohol. Sufficient ether mixture is added at room temperature so that the final weight of the trinitroglycerol plus polyvinylalcohol provides a 10% trinitroglycerol and 90% polyvinylalcohol mixture. After addition is complete, stirring of the mixture is conducted until total evaporation of the ether, yielding a white powder. This white powder is free flowing and in physical appearance is similar to that of the starting polyvinylalcohol; it is soluble in water and the binding of the polyvinylalcohol to trinitroglycerol is shown by the maintenance of such property after centrifugation at 5,000 g for 30 minutes. The operation of evaporation is advantageously conducted under a hood.

Example II

Acetone is substituted for ether in Example I, the result being the formation of the same white powdery substance.

Example III

Substituting polyvinylpyrrolidone (mw 40,000) for the polyvinylalcohol of Example I yields a powdery triturate which has the trinitroglycerol firmly bound to the polymer.

While the triturate form may be used for the preparation of the polymeric diffusion matrix of the invention, it is also contemplated that the trinitroglycerol may be directed added to the mixture in a solution form, bearing in mind the importance of avoidance of phase separation and the explosive consequences which may result from such separation.

Example IV

Lactose triturate (10% trinitroglycerol) is added to sufficient acetone to put the trinitroglycerol in solution, after which the acetone solution is admixed with polyethylene glycol in an amount sufficient to yield an eventual trinitroglycerol concentration in a solution thereof of 18% (exclusive of the weight of the acetone). The lactose is then separated from the solution. The resultant solution is thereafter treated to heating under vacuum to yield the acetone-free trinitroglycerol solvent mixture that may be used for the introduction of the trinitroglycerol in a substantially disaccharide-free form to prepare the polymeric diffusion matrix of the invention.

Example V

Lactose triturate (10% trinitroglycerol) is added to sufficient acetone to put the trinitroglycerol in solution, after which the acetone solution is admixed with dipropylene glycol in an amount sufficient to yield an eventual trinitroglycerol concentration in a solution thereof of 6% (exclusive of the weight of the acetone). The lactose is then separated from the solution. The resultant solution is thereafter treated to heating under vacuum to yield the acetone-free trinitroglycerol solvent mixture that may be used for the introduction of the trinitroglycerol in a substantially disaccharide-free form to prepare the polymeric diffusion matrix of the invention.

Example VI

Lactose triturate (10% trinitroglycerol) is added

to sufficient acetone to put the trinitroglycerol in solution, after which the acetone solution is admixed with diacetin in an amount sufficient to yield an eventual trinitroglycerol concentration in a solution thereof of 6% (exclusive of the weight of the acetone). The lactose is then separated from the solution. The resultant solution is thereafter treated to heating under vacuum to yield the acetone-free trinitroglycerol solvent mixture that may be used for the introduction of the trinitroglycerol in a substantially disaccharide-free form to prepare the polymeric diffusion matrix of the invention.

Example VII

Lactose triturate (10% trinitroglycerol) is added to sufficient acetone to put the trinitroglycerol in solution, after which the acetone solution is admixed with acetin in an amount sufficient to yield an eventual trinitroglycerol concentration in a solution thereof of 6% (exclusive of the weight of the acetone). The lactose is then separated from the solution. The resultant solution is thereafter treated to heating under vacuum to yield the acetone-free trinitroglycerol solvent mixture that may be used for the introduction of the trinitroglycerol in a substantially disaccharide-free form to prepare the polymeric diffusion matrix of the invention.

Example VIII

Lactose triturate (10% trinitroglycerol) is added to sufficient acetone to put the trinitroglycerol in solution, after which the acetone solution is admixed with propylene glycol (molecular weight 200, PEG 200) in an amount sufficient to yield an eventual trinitroglycerol concentration in a solution thereof of 6% (exclusive of the weight of the acetone). The lactose is then separated from the solution. The resultant solution is thereafter treated to heating under vacuum to yield the acetone-free trinitroglycerol solvent mixture that may be used for the introduction of the trinitroglycerol in a substantially disaccharide-free form to prepare the polymeric diffusion matrix of the invention.

The percentages given in the following examples express an amount by weight of the individual component in the final polymeric diffusion matrix.

Example IX

Glycerol, at standard 96% concentration, is added to a vessel in an amount to prepare an 8 percent composition. Also added to the same vessel is sufficient Monamid 716P (Mona) to yield a final product with 5.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 26%. Water is added in an amount

to yield a total final composition weight of 38 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example X

Glycerol, at standard 96% concentration, is added to a vessel in an amount to prepare a 14 percent composition. A first high molecular weight polyvinylalcohol component (mw 115,000; Elvano® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XI

Glycerol, at standard 96% concentration, is added to a vessel in an amount to prepare a 13.5 percent composition. Also added to the same vessel is sufficient Monamid 716P (Mona) to yield a final product with 0.5 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XII

Glycerol, at standard 96% concentration, is added to a vessel in an amount to prepare a 13 percent composition. Also added to the same vessel is sufficient Monamid 716P (Mona) to yield a final product with 1.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol, with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resulting liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XIII

Glycerol, at standard 96% concentration, is added to a vessel in an amount to prepare a 12 percent composition. Also added to the same vessel is sufficient Monamid 716P (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XIV

Glycerol, at standard 96% concentration, is added to a vessel in an amount to prepare a 13 percent composition. Also added to the same vessel is sufficient Monateric LMAB (Mona) to yield a final product with 1.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XV

Glycerol, at standard 96% concentration, is added to a vessel in an amount to prepare a 12 percent composition. Also added to the same vessel is sufficient Monateric LMAB (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XVI

Glycerol, at standard 96% concentration, is added to a vessel in an amount to prepare a 11 percent composition. Also added to the same vessel is sufficient Monateric LMAB (Mona) to yield a final product with 3.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed.

The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XVII

Glycerol, at standard 96% concentration, is added to a vessel in an amount to prepare a 13 percent composition. Also added to the same vessel is sufficient Monaquat PTL (Mona) to yield a final product with 1.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XVIII

Glycerol, at standard 96% concentration, is added to a vessel in an amount to prepare a 12 percent composition. Also added to the same vessel is sufficient Monaquat PTL (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resultant liquid matrix is then cast between two sheets of a foil laminate, allowed to set, yielding the desired polymeric matrix not suitable for use as a transdermal system for the sustained release of trinitroglycerol.

The matrix is strong and exhibits only slight surface wetness.

Example XIX

Glycerol, at standard 96% concentration, is added to a vessel in an amount to prepare a 11 percent composition. Also added to the same vessel is sufficient Monaquat PTL (Mona) to yield a final product with 3.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example V is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XX

Glycerol, at standard 96% concentration, is added to a vessel in an amount to prepare a 14 percent composition. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VI is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXI

Glycerol, at standard 96% concentration, is added to a vessel in an amount to prepare a 13 percent composition. Also added to the same vessel is sufficient Monamid 716P (Mona) to yield a final product with 1.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VI is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXII

Glycerol, at standard 96% concentration, is added to a vessel in an amount to prepare a 12 percent composition. Also added to the same vessel is sufficient Monamid 716P (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VI is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXIII

Glycerol, at standard 96% concentration, is added to a vessel in an amount to prepare a 11 percent composition. Also added to the same vessel is sufficient Monamid 716P (Mona) to yield a final product with 3.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VI is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXIV

Glycerol, at standard 96% concentration, is added to a vessel in an amount to prepare a 12 percent composition. Also added to the same vessel is sufficient Monamid 716 (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VI is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXV

Glycerol, at standard 96% concentration, is added to a vessel in an amount to prepare a 12 percent composition. Also added to the same vessel is sufficient Monateric LMAB (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VI is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXVI

Glycerol, at standard 96% concentration, is added to a vessel in an amount to prepare a 12 percent composition. Also added to the same vessel is sufficient Monaquat PTL (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VI is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXVII

Glycerol, at standard 96% concentration, is added to a vessel in an amount to prepare a 14 percent composition. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXVIII

Glycerol, at standard 96% concentration, is added to a vessel in an amount to prepare a 13 percent composition. Also added to the same vessel is sufficient Monateric LMAB (Mona) to yield a final product with 1.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXIX

Glycerol, at standard 96% concentration, is added to a vessel in an amount to prepare a 12 percent composition. Also added to the same vessel is sufficient Monateric LMAB (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXX

Glycerol, at standard 96% concentration, is added to a vessel in an amount to prepare a 11 percent composition. Also added to the same vessel is sufficient Monateric LMAB (Mona) to yield a final product with 3.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol 4-98®, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXXI

Glycerol, at standard 96% concentration, is added to a vessel in an amount to prepare a 12 percent composition. Also added to the same vessel is sufficient Monamid 716P (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXXII

Glycerol, at standard 96% concentration, is added to a vessel in an amount to prepare a 12 percent composition. Also added to the same vessel is sufficient Monateric LMAB (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXXIII

Glycerol, at standard 96% concentration, is added to a vessel in an amount to prepare a 12 percent composition. Also added to the same vessel is sufficient Monaquat PTL (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol 4-98®, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXXIV

Glycerol, at standard 96% concentration, is added to a vessel in an amount to prepare a 13 percent composition. Also added to the same vessel is sufficient Monateric LMAB (Mona) to yield a final product with 1.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount

to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXXV

Glycerol, at standard 96% concentration, is added to a vessel in an amount to prepare a 11 percent composition. Also added to the same vessel is sufficient Monateric LMAB (Mona) to yield a final product with 3.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXXVI

Glycerol, at standard 96% concentration, is added to a vessel in an amount to prepare a 13 percent composition. Also added to the same vessel is sufficient Monateric LMAB (Mona) to yield a final product with 1.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXXVII

Glycerol, at standard 96% concentration, is added to a vessel in an amount to prepare a 11 percent composition. Also added to the same vessel is sufficient Monaquat PTL (Mona) to yield a final product with 3.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXXVIII

Glycerol, at standard 96% concentration, is added to a vessel in an amount to prepare a 12 percent composition. Also added to the same vessel is sufficient Monamid 716P (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VIII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XXXIX

Glycerol, at standard 96% concentration, is added to a vessel in an amount to prepare a 12 percent composition. Also added to the same vessel is sufficient Monateric LMAB (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VIII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XL

Glycerol, at standard 96% concentration, is added to a vessel in an amount to prepare a 12 percent composition. Also added to the same vessel is sufficient Monateric LMAB (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VIII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XLI

Glycerol, at standard 96% concentration, is added to a vessel in an amount to prepare a 12 percent composition. Also added to the same vessel is sufficient Monaquat PTL (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 28%. Water is added in an amount to yield a total final composition weight of 47 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example VIII is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XLII

Glycerol, at standard 96% concentration, is added to a vessel in an amount to prepare a 8 percent composition. Also added to the same vessel is sufficient Monamid 716P (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 26%. Water is added in an amount to yield a total final composition weight of 41 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XLIII

Glycerol, at standard 96% concentration is, added to a vessel in an amount to prepare a 8 percent composition. Also added to the same vessel is sufficient Monateric LMAB (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 26%. Water is added in an amount to yield a total final composition weight of 41 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XLIV

Glycerol, at standard 96% concentration, is added to a vessel in an amount to prepare a 8 percent composition. Also added to the same vessel is sufficient Monaquat PTL (Mona) to yield a final product with 2.0 percent thereof. A first high molecular weight polyvinylalcohol component (mw 115,000; Elvanol® 71-30, DuPont) is added to yield 3% of the final product. The second component, a water-soluble polymer with hydration sites, polyvinylalcohol with a molecular weight of 20,000 (Mowiol® 4-98, Hoechst) is added to yield a final percentage composition of 26%. Water is added in an amount to yield a total final composition weight of 41 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface wetness.

Example XLV

Glycerol, at standard 96% concentration, is added to a vessel in an amount to prepare a 10 percent composition. Also added to the same vessel is sufficient Monamid 716P (Mona) to yield a final product with 3 percent thereof. A first high molecular weight polyvinylalcohol component (mw 115,000; Elvanol® 71-30, DuPont) is added to yield 1.0% of the final product. The second component, a water-soluble polymer with hydration sites, polyvinylalcohol with a molecular weight of 20,000 (Mowiol® 4-98, Hoechst) is added to yield a final percentage composition of 27%. Polyvinylpyrrolidone of molecular weight 40,000 is added to a final amount of 1.5 percent. Water is added in an amount to yield a total final composition weight of 37.5 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is not as suitable as the preferred embodiment, since it is moderately wet and brittle.

Example XLVI

Glycerol, at standard 96% concentration, is added to a vessel in an amount to prepare a 5 percent composition. Also added to the same vessel is sufficient Monamid 716P (Mona) to yield a final product with 3 percent thereof. A first high molecular weight polyvinylalcohol component (mw 115,000; Elvanol® 71-30, DuPont) is added to yield 0.4% of the final product. The second component, a water-soluble polymer with hydration sites, polyvinylalcohol with a molecular weight of 20,000 (Mowiol® 4-98, Hoechst) is added to yield a final percentage composition of 25%. Polyvinylpyrrolidone of molecular weight 40,000 is added to a final amount of 1.6 percent. Water is added in an amount to yield a total final composition weight of 45 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is not as suitable as the preferred embodiment, since it is brittle although only slightly damp.

Example XLVII

Glycerol, at standard 96% concentration, is added to a vessel in an amount to prepare a 5 percent composition. Also added to the same vessel is sufficient Monamid 716P (Mona) to yield a final product with 3 percent thereof. A first high molecular weight polyvinylalcohol component (mw 115,000; Elvanol® 71-30, DuPont) is added to yield 1.0% of the final product. The second component, a water-soluble polymer with hydration sites, polyvinylalcohol with a molecular weight of 20,000 (Mowiol® 4-98, Hoechst) is added to yield a final percentage composition of 23%. Polyvinylpyrrolidone of molecular weight 40,000 is added to a final amount of 1.5 percent. Water is added in an amount to yield a total final composition weight of 46.5 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is not as suitable as the preferred embodiment, since it is brittle although only slightly damp.

Example XLVIII

Glycerol, at standard 96% concentration, is added to a vessel in an amount to prepare a 11 percent composition. Also added to the same vessel is sufficient Monamid 716P (Mona) to yield a final product with 3 percent thereof. A first high molecular weight polyvinylalcohol component (mw 115,000; Elvanol® 71-30, DuPont) is added to yield 3.0% of the final product. The second component, a water-soluble polymer with hydration sites, polyvinylalcohol with a molecular weight of 20,000 (Mowiol® 4-98, Hoechst) is added to yield a final percentage composition of 22%. Polyvinylpyrrolidone of molecular weight 40,000 is added to a final amount of 4.0 percent. Water is added in an amount to yield a total final composition weight of 38 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is not as suitable as the preferred embodiment, since it is too wet although strong.

Example XLIX

Glycerol, at standard 96% concentration, is added to a vessel in an amount to prepare a 7 percent composition. Also added to the same vessel is sufficient Monamid 716P (Mona) to yield a final product with 3 percent thereof. A first high molecular weight polyvinylalcohol component (mw 115,000; Elvanol® 71-30, DuPont) is added to yield 3.0% of the final product. The second component, a water-soluble polymer with hydration sites, polyvinylalcohol with a molecular weight of 20,000 (Mowiol® 4-98, Hoechst) is added to yield a final percentage composition of 22%. Polyvinylpyrrolidone of molecular weight 40,000 is added to a final amount of 0.5 percent. Water is added in an amount to yield a total final composition weight of 44.5 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is strong and exhibits only slight surface dampness.

Example L

Glycerol, at standard 96% concentration, is added to a vessel in an amount to prepare a 8 percent composition. Also added to the same vessel is sufficient Monamid 716P (Mona) to yield a final product with 5 percent thereof. A first high molecular weight polyvinylalcohol component (mw 115,000; Elvanol® 71-30, DuPont) is added to yield 3.0% of the final product. The second component, a water-soluble polymer with hydration sites, polyvinylalcohol with a molecular weight of 20,000 (Mowiol® 4-98, Hoechst) is added to yield a final percentage composition of 22%. Polyvinylpyrrolidone of molecular weight 40,000 is added to a final amount of 4.0 percent. Water is added in an amount to yield a total final composition weight of 38 percent (deionized water is used in the experiments). After each of the mentioned components has been added to the vessel, which typically may be a beaker for experimental studies, the components are thoroughly mixed. The resultant mixture is placed in a microwave oven and heated to the boiling point. The beaker is then covered and thereafter transferred to a steam bath and the process continued until full extension of the polymers contained in the mixture.

A trinitroglycerol solution prepared in accordance with Example IV is added to the above mixture stirring and mixed well. The resultant liquid matrix is then cast between two sheets of a foil laminate, allowed to set, yielding the desired polymeric matrix now suitable for use as a transdermal system for the sustained release of trinitroglycerol.

The matrix is not as suitable as the preferred embodiment, since it is too wet, although strong.

Examples LI—XCII
(44 examples)

Substituting the triturate of Example I for the trinitroglycerol solutions of Examples IX—L, polymeric diffusion matrices free from disaccharides are produced that may be used as a polymeric diffusion matrix in accordance with the invention.

**Claims**

1. A trinitroglycerol containing polymeric diffusion matrix for the transdermal systemic delivery of trinitroglycerol through the skin of a patient, said polymeric diffusion matrix containing sufficient trinitroglycerol to be released over a prolonged period of time, characterized in that it is substantially disaccharide-free and comprises 10 to 40 percent by weight of a first lower molecular weight, partial hydrolyzed polyvinylalcohol component with a molecular weight of from 5.000 to 40.000 and with a degree of hydrolysis of 75 to 92 percent and 2 to 15 percent by weight of a second higher molecular weight, essentially fully hydrolyzed polyvinylalcohol component with a molecular weight of from 90.000 to 150.000 and with a degree of hydrolysis of at least 95 percent and 2 to 25 percent by weight of glycerol.

2. The polymeric diffusion matrix of claim 1 which includes polyvinylpyrrolidone in an amount of up to 2 percent by weight and which has a molecular weight of from 20.000 to 60.000.

3. The polymeric diffusion matrix of claim 1 which includes an alkanolamide which is present in an amount of up to 5%.

4. The polymeric diffusion matrix of claim 3, wherein said alkanolamide is present in an amount of from 0.1 to 3% by weight.

5. The polymeric diffusion matrix of claim 1, 2, 3 or 4, which includes a backing layer adhered to one surface of said polymeric diffusion matrix, said backing layer having a larger surface area than said polymeric diffusion matrix, and an adhesive layer attached to said backing layer on the same side thereof as said polymeric diffusion matrix, whereby said polymeric diffusion matrix is maintained in diffusional contact with the skin of said patient.

6. The polymeric diffusion matrix of claims 1, 2, 3, 4 or 5, which includes a facestock layer with skin adhesive in said polymeric matrix which permits a self-adhesion of said polymeric matrix to the skin of said patient.

**Patentansprüche**

1. Trinitroglycerin enthaltende polymere Diffusionsmatrix zur transdermalen systemischen Verabreichung von Trinitroglycerin durch die Haut eines Patienten, wobei die polymere Diffusionsmatrix genügend Trinitroglycerin enthält, damit dieses während einer längeren Zeitspanne abgegeben werden kann, dadurch gekennzeichnet, daß sie im wesentlichen frei von Disaccharid ist und 10 bis 40 Gew.-% einer ersten, niedermolekularen, partiell hydrolysierten Polyvinylalkohol-Komponente mit einem Molekulargewicht von 5 000 bis 40 000 und einem Hydrolysegrad von 75 bis 92% und 2 bis 15 Gew.-% einer zweiten, höhermolekularen, im wesentlichen vollständig hydrolysierten Polyvinylalkohol-Komponente mit einem Molekulargewicht von 90 000 bis 150 000 und einem Hydrolysegrad von wenigstens 95% sowie 2 bis 25 Gew.-% Glycerin enthält.

2. Polymere Diffusionsmatrix nach Anspruch 1, dadurch gekennzeichnet, daß sie Polyvinylpyrrolidon in einer Menge bis zu 2 Gew.-% und mit einem Molekulargewicht von 20 000 bis 60 000 enthält.

3. Polymere Diffusionmatrix nach Anspruch 1, dadurch gekennzeichnet, daß sie ein Alkanolamid enthält, das in einer Menge bis zu 5% vorhanden ist.

4. Polymere Diffusionsmatrix nach Anspruch 3, dadurch gekennzeichnet, daß das Alkanolamid in einer Menge von 0,1 bis 3 Gew.-% vorhanden ist.

5. Polymere Diffusionsmatrix nach Ansprüchen 1, 2, 3 oder 4, dadurch gekennzeichnet, daß sie eine auf der einen Oberfläche der polymeren Diffusionsmatrix aufgebrachte Kaschierschicht, die eine größere Oberfläche als die polymere Diffusionsmatrix aufweist, sowie eine an der Kaschierschicht auf der gleichen Seite wie die polymere Diffusionsmatrix aufgebrachte Klebeschicht umfaßt, durch welche die polymere Diffusionsmatrix in Diffusionsberührung mit der Haut des Patienten gehalten wird.

6. Polymere Diffusionsmatrix nach Ansprüchen 1, 2, 3, 4 oder 5, dadurch gekennzeichnet, daß sie ein stirnseitiges Schichtmaterial mit einem Haut-Klebstoff in der polymeren Matrix enthält, die ein Selbsthaften der polymeren Matrix an der Haut des Patienten ermöglicht.

**Revendications**

1. Matrice de diffusion polymérique contenant du trinitroglycérol pour une administration systémique transdermique de trinitroglycérol à travers la peau d'un malade, ladite matrice polymérique de diffusion contenant une quantité suffisante de trinitroglycérol à être délivrée sur une période de temps prolongée, caractérisée par le fait qu'elle est essentiellement exempte de disaccharide et comprend de 10 à 40% en poids d'un premier composant d'un alcool polyvinylique partiellement hydrolysé de faible poids moléculaire, ayant un poids moléculaire de 5.000 à 40.000 et un degré d'hydrolyse de 75 à 92% et de 2 à 15% en poids d'un second composant d'alcool polyvinylique essentiellement totalement hydrolysé de faible poids moléculaire élevé, avec un poids moléculaire de 90.000 à 150.000 et avec un degré d'hydrolyse d'au moins 95%, et de 2 à 25% en poids de glycérol.

2. Matrice de diffusion polymérique selon la revendication 1, comprenant de la polyvinylpyrrolidone en une quantité maximum de 2% en poids et ayant un poids moléculaire de 20.000 à 60.000.

3. Matrice de diffusion polymérique selon la revendication 1, qui comprend un alkanolamide présent en une quantité maximum de 5%.

4. Matrice de diffusion polymérique selon la revendication 3, dans laquelle ledit alkanolamide est présent en une quantité de 0,1 à 3% en poids.

5. Matrice de diffusion polymérique selon les revendications 1, 2, 3 ou 4, qui comprend une couche de soutien fixée sur une surface de ladite matrice de diffusion polymérique, ladite couche de soutien ayant une zone de surface plus large que ladite matrice de diffusion polymérique et une couche d'adhésif fixée sur ladite couche de soutien sur le même côté que ladite matrice de diffusion polymérique, ce par quoi ladite matrice de diffusion polymérique est maintenue en contact de diffusion avec la peau dudit patient.

6. Matrice de diffusion polymérique selon les revendications 1, 2, 3, 4 ou 5, qui comprend une couche comportant un adhésif pour la peau sur ladite matrice polymérique, ce qui permet une auto-adhésion de ladite matrice polymérique sur la peau dudit patient.